# FASCICULE DE BREVET EUROPEEN

(11) **EP 2 728 999 B1**
(45) Date de publication et mention de la délivrance du brevet: **03.02.2016**
(21) Numéro de dépôt: 12738556.5
(22) Date de dépôt: 03.07.2012
(51) Int. Cl.: A01N 1/02, A61J 1/16, A61J 1/10, A61J 1/14

(54) **KIT POUR LA CONSERVATION D'UN PRODUIT BIOLOGIQUE COMPRENANT UNE POCHE TRIDIMENSIONNELLE ET UNE ENVELOPPE TRIDIMENSIONNELLE ADAPTÉE**
KIT ZUR KONSERVIERUNG EINES BIOLOGISCHEN PRODUKTS MIT EINEM DREIDIMENSIONALEN BEUTEL UND EINER ZUGEHÖRIGEN DREIDIMENSIONALEN HÜLLE
KIT FOR PRESERVING A BIOLOGICAL PRODUCT INCLUDING A THREE-DIMENSIONAL BAG AND A MATCHING THREE-DIMENSIONAL OVERWRAP

(30) Priorité: 08.07.2011 FR 1156212
(43) Date de publication de la demande: 14.05.2014
(73) Titulaire: MACO PHARMA, 59420 Mouvaux (FR)
(72) Inventeur: DELORME, Bruno, F-59700 Marcq-en-Baroeul (FR); PLAINFOSSE, Marie, F-59700 Marcq-en-Baroeul (FR)
(74) Mandataire: Sayettat, Julien Christian
(86) Numéro de dépôt international: PCT/FR2012/051553
(87) Numéro de publication internationale: WO 2013/007921

(56) Documents cités:
- EP-A2- 0 253 651
- EP-A2- 1 627 566
- FR-A1- 2 717 782
- US-A1- 2005 084 838
- US-B1- 6 232 115
- RUBINSTEIN ET AL: "Why Cord Blood?", HUMAN IMMUNOLOGY, vol. 67, no. 6, 1 juin 2006 (2006-06-01), pages 398-404, XP024993240, ISSN: 0198-8859, DOI: 10.1016/J.HUMIMM.2006.03.015 [extrait le 2006-06-01] -& "Cord Blood Collection, Processing and Freezing Bag Sets for the Cryopreservation of Blood and Cellular Components (Pall Medical)", , 1 janvier 2009 (2009-01-01), XP055016286, Extrait de l'Internet: URL:http://www.pall.com/pdfs/Medical/09268 0_CordBld_BagSes_SS.pdf [extrait le 2012-01-12] -& "BioArchive System Acessories", , 1 janvier 2008 (2008-01-01), XP055016284, Extrait de l'Internet: URL:http://www.thermogenesis.com/CMSFiles/ Pdf/Technical/BAaccessSS.pdf [extrait le 2012-01-12]
- John K. Fraser ET AL: "Cord Blood Transplantation Study (COBLT): Cord Blood Bank Standard Operating Procedures", Journal of Hematotherapy, 1 janvier 1998 (1998-01-01), pages 521-561, XP055016461, Extrait de l'Internet: URL:http://www.liebertonline.com/doi/pdf/1 0.1089/scd.1.1998.7.521 [extrait le 2012-01-13]
- L. ABBRUZZESE ET AL: "A new freezing and storage procedure improves safety and viability of haematopoietic stem cells and neutrophil engraftment: a single institution experience", VOX SANGUINIS, vol. 98, no. 2, 1 février 2010 (2010-02-01), pages 172-180, XP055016458, ISSN: 0042-9007, DOI: 10.1111/j.1423-0410.2009.01239.x

## Description

L'invention concerne un kit pour la conservation d'un produit biologique comprenant une poche tridimensionnelle et une enveloppe destinée à loger ladite poche.

L'invention s'applique de façon générale à la conservation de produits liquides ou semi liquides biologiques tels que le sang, les composants sanguins, les cellules, ou les tissus biologiques. L'invention s'applique notamment à la cryoconservation du sang de cordon ombilical, de vaccins ou de milieux de culture cellulaire conditionnés.

Le sang de cordon ombilical renferme un grand nombre de cellules souches, notamment des cellules souches hématopoïétiques qui sont à l'origine des différentes cellules du sang : les globules rouges, les globules blancs et les plaquettes. Ces cellules participent à la défense de l'organisme (système immunitaire). Ainsi et depuis la première greffe de sang de cordon en 1988, la greffe de sang de cordon est devenue une alternative à la greffe de moelle osseuse et elle est de plus en plus utilisée. De plus, la présence dans le sang de cordon d'autres cellules d'intérêt, telles que les cellules souches mésenchymateuses, les cellules souches multipotentes, et des cellules du système immunitaire, ouvre la voie à d'autres applications tant en médecine régénérative (traitement du diabète, de l'infarctus du myocarde, des maladies neurodégénératives...) qu'en immunothérapie.

Pour répondre au besoin croissant en sang de cordon ombilical, des banques destinées à conserver ce sang ont été créées. Après le prélèvement d'une unité de sang de cordon à l'aide d'un système à poches tel que décrit dans le document EP-1 262 202, et après une étape dite de "réduction de volume", l'unité de sang de cordon ainsi traitée est transférée dans une poche de congélation. La poche de congélation est ensuite placée dans une cassette métallique qui est finalement plongée dans l'azote liquide pour conservation. En variante, la poche est plongée directement dans l'azote liquide.

Des poches tridimensionnelles pouvant résister à des températures extrêmes et permettant une congélation / décongélation uniforme afin de ne pas endommager les cellules ont été développées. De telles poches sont décrites dans les documents WO-97/49959, WO-2004/108057 ou WO-98/09872.

Aujourd'hui, pour éviter les risques de fuite de sang de cordon pendant leur conservation et empêcher des contaminations croisées entre plusieurs unités de sang de cordon conservées dans un même container d'azote liquide, la poche de congélation est d'abord placée dans une enveloppe fermée hermétiquement avant d'être éventuellement placée dans la cassette métallique. Ainsi, si une poche se perce, le sang s'écoule dans l'enveloppe hermétique sans souiller les autres poches. Cette procédure est par exemple décrite dans le document WO 2010/119311.

Une enveloppe pour protéger une poche de conservation est décrite dans le document EP-A1-1 864 641. L'enveloppe est constituée d'un film relativement fin comprenant une couche adhésive d'un fluoropolymère.

L'article de P. Rubinstein, "Why Cord Blood? ", Human Immunology, 2006, vol. 67, p. 398-404 décrit l'utilisation d'une enveloppe bidimensionelle de Teflon pour protéger les poches tridimensionnelles de congélation.

Ce type d'enveloppes bidimensionnelles est mal adapté aux poches de conservation du sang de cordon. En premier lieu, il n'est pas facile de glisser les poches de conservation dans ces enveloppes dont les dimensions sont ajustées aux dimensions des poches.

Ensuite, l'enveloppe crée une épaisseur supplémentaire de sorte qu'il n'est pas facile de placer l'ensemble poche et enveloppe dans une cassette métallique.

Enfin, avant la fermeture de l'enveloppe, il est nécessaire de procéder à l'élimination ou la réduction de la quantité d'air retenue entre la poche et l'enveloppe. En effet, cette quantité d'air peut gêner la congélation et la décongélation de l'unité de sang de cordon et provoquer des dommages aux cellules d'intérêts contenues dans le sang de cordon. De plus, et afin d'optimiser au maximum les espaces de stockage, les cassettes métalliques peuvent posséder des dimensions ajustées à celle des poches de congélation. Dès lors, une trop grande quantité d'air augmente le volume de l'ensemble poche et enveloppe, ce qui peut encore conduire à gêner la mise en place de la poche de congélation dans la cassette métallique.

L'invention propose un kit comprenant une poche de conservation et une enveloppe adaptée permettant d'insérer plus facilement la poche dans son enveloppe. L'ensemble poche et enveloppe est ensuite facilement placé dans une cassette métallique pour congélation.

A cet effet l'invention propose un kit pour la conservation d'un produit biologique comprenant d'une part une poche destinée à contenir ledit produit biologique, ladite poche possédant une géométrie tridimensionnelle sensiblement constante lors de son utilisation, et d'autre part une enveloppe destinée à emballer ladite poche, ladite enveloppe comprenant un logement agencé pour recevoir ladite poche, ledit logement possédant une géométrie tridimensionnelle sensiblement constante lors de son utilisation, et une fente étant prévue le long d'un bord dudit logement pour permettre l'insertion de la poche dans ledit logement, une bande antérieure et une bande postérieure s'étendent extérieurement à partir de ladite fente, lesdites bandes étant destinées à être associées entre elles pour fermer hermétiquement ledit logement de l'enveloppe.

D'autres objets et avantages apparaîtront au cours de la description qui suit.
La figure 1 représente de façon schématique une vue de face d'une poche tridimensionnelle munie d'une tubulure et de connecteurs.
La figure 2 représente de façon schématique une vue en coupe selon la ligne A-A de la poche de la figure 1.
La figure 3 représente de façon schématique une vue en perspective de l'enveloppe selon une réalisation particulière.
La figure 4 représente de façon schématique une vue de côté de l'enveloppe de la figure 3.
Les figures 5 et 6 représentent de façon schématique une vue de dessus et de dessous respectivement, et en perspective, d'une des feuilles thermoformées constituant l'enveloppe de la figure 3.
La figure 7 représente de façon schématique une vue en perspective de la poche de la figure 1 placée dans l'enveloppe de la figure 3.
La figure 8 représente de façon schématique une vue en perspective d'une feuille d'une enveloppe selon une autre réalisation particulière.

L'invention concerne un kit pour la conservation d'un produit biologique comprenant d'une part une poche destinée à contenir ledit produit biologique et d'autre part une enveloppe destinée à emballer ladite poche.

Un produit biologique est par exemple du sang, un composant sanguin tel que du sérum, du plasma, des plaquettes, des globules blancs, des globules rouges, un buffy coat, un lysat plaquettaire, ou de la moelle osseuse.

D'autres exemples de produits biologiques sont des produits comprenant des enzymes comme la trypsine, des lignées cellulaires, des cellules associées à des matrices de type nano-, macro-matrices ou mini billes, des surnageants d'intérêt de produits cellulaires, ou des vaccins.

Les produits biologiques comprennent également les milieux de culture, conditionnés ou non, les solutions de conservation d'organes, tissus et/ou cellules, et les substituts sanguins (hémoglobine synthétique) et les molécules biopharmaceutiques.

Notamment, le produit biologique comprend des cellules humaines ou animales, en particulier des cellules à usage thérapeutique telles que des cellules souches.

Plus spécifiquement, le produit biologique à conserver est une unité réduite de sang de cordon, c'est-à-dire un volume de sang de cordon prélevé, après la naissance du bébé, d'un cordon ombilical, et qui a été traité par exemple par centrifugation et/ou ajout d'amidon (HES, hydroxyethylstarch) afin d'obtenir un plus petit volume de sang de cordon dépourvu de globules rouges et enrichi en cellules d'intérêts.

Dans une variante, le produit biologique est additionné d'un additif, tel qu'un agent cryoprotecteur de type DMSO (diméthyl sulfoxyde) et/ou glycérol.

La conservation du produit biologique est réalisée à diverses températures (4°C, -20°C, -80°C) et notamment par congélation du produit biologique puis stockage dans de l'azote liquide à -196°C.

Pour conserver un produit biologique de type sang de cordon, il est connu d'utiliser des poches tridimensionnelles réalisées par moulage, comme décrit dans les documents brevets cités plus haut.

Contrairement aux poches bidimensionnelles, réalisées par exemple à partir de deux feuilles substantiellement planes reliées entre elles sur leur périphérie ou d'une gaine dont les extrémités sont fermées, qui, lorsqu'elles sont vides, sont substantiellement plates, les poches tridimensionnelles vides ne sont pas plates et possèdent un certain relief.

Selon l'invention, la poche du kit possède une géométrie tridimensionnelle sensiblement constante lors de son utilisation.

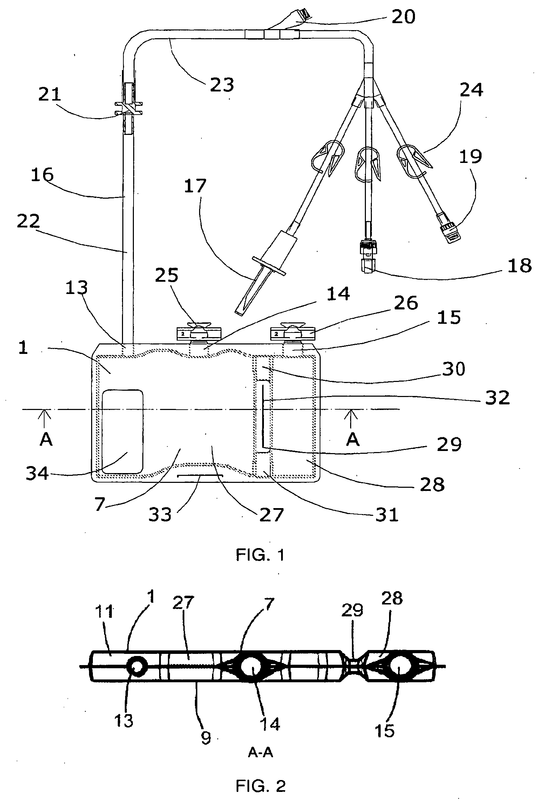

Une telle poche est représentée sur les figures 1 et 2. La poche 1 possède une géométrie tridimensionnelle sensiblement constante lors de son utilisation, c'est-à-dire notamment que la poche ne gonfle pas lorsqu'on la remplit d'un liquide. La géométrie de la poche est fixée au moment de la fabrication en donnant une forme prédéfinie à la poche. La poche conserve sa géométrie d'origine lors de son remplissage avec le produit biologique à conserver et lors de son stockage.

Sur les figures 1 et 2, la poche 1 possède sensiblement la forme d'un parallélépipède rectangle, c'est-à-dire un parallélépipède dont les 6 faces sont des rectangles.

Contrairement à une poche bidimensionnelle qui change de géométrie à mesure qu'elle est remplie d'un liquide, les géométries de la poche tridimensionnelle vide et remplie du produit biologique à conserver sont sensiblement identiques.

Lors de la conservation de la poche, il est avantageux de suremballer ladite poche dans une enveloppe 2 et de la fermer hermétiquement afin d'éviter qu'une fuite dans une poche produise une contamination des autres poches stockées dans son voisinage.

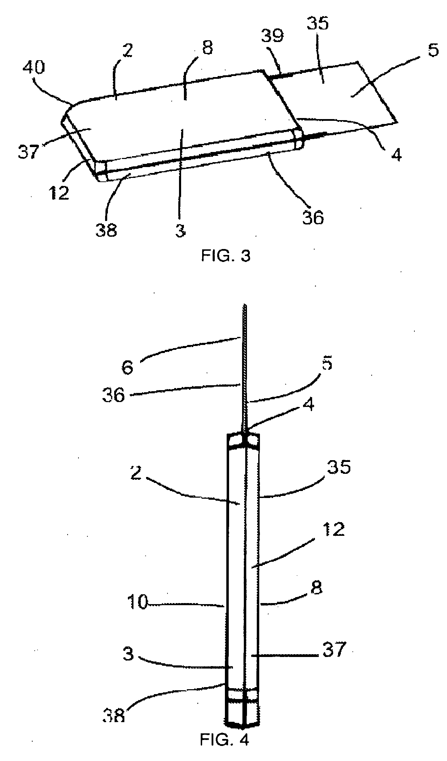

Selon l'invention et comme illustré sur la figure 3, l'enveloppe 2 destinée à emballer ladite poche comprend un logement agencé pour recevoir ladite poche 1. Ce logement possède une géométrie tridimensionnelle sensiblement constante lors de son utilisation, c'est-à-dire que le logement n'est pas déformé lorsqu'on y insère la poche. Le logement de l'enveloppe possède une géométrie sensiblement identique lorsqu'il est vide ou lorsqu'il contient la poche. Cette géométrie est déterminée au moment de la fabrication, en donnant une forme prédéfinie au logement.

Une fente 4 est prévue le long d'un bord dudit logement 3 pour permettre l'insertion de la poche dans ledit logement, une bande antérieure 5 et une bande postérieure 6 s'étendent extérieurement à partir de ladite fente 4. Ces bandes 5,6 sont destinées à être associées entre elles pour fermer hermétiquement ledit logement 3 de l'enveloppe 2, une fois la poche placée dans ledit logement 3 de l'enveloppe 2.

Ces bandes 5,6 permettent en outre d'ouvrir plus facilement la fente 4 de l'enveloppe pour faciliter l'insertion de la poche dans son logement 3.

Une fois la poche placée dans le logement 3, les bandes 5,6 sont associées entre elles, notamment par soudure, pour fermer la fente 4. Les bandes 5,6 sont ensuite séparées de l'enveloppe 2.

Les dimensions intérieures de la géométrie du logement 3 correspondent sensiblement aux dimensions extérieures de la géométrie de ladite poche 1 à placer dans le logement 3, afin de minimiser l'espace, et donc le volume d'air, entre l'enveloppe 2 et la poche 1 une fois la poche logée dans le logement.

En effet, la présence d'air gêne le bon déroulement de la congélation et de la décongélation de la poche et peut entraîner l'endommagement des cellules d'intérêts. Avec des dimensions ainsi ajustées, le volume d'air entre la poche et son logement est minimal, ce qui permet d'optimiser la conservation du produit biologique.

En outre, avec des dimensions enveloppe/poche ainsi adaptées, la présence de l'enveloppe autour de la poche n'a pas d'impact important sur l'espace occupé par l'ensemble formé par l'enveloppe et la poche. En effet, la présence de l'enveloppe ne gêne pas l'insertion de la poche dans une cassette métallique de congélation.

Structurellement, la poche 1 et le logement 3 sont formés respectivement d'une face antérieure 7,8 et d'une face postérieure 9,10 sensiblement planes, reliées entre elles par des parois latérales 11,12, chacune desdites faces et desdites parois de la poche et dudit logement étant destinées à être disposées en regard l'une de l'autre.

La fente 4 de l'enveloppe est prévue sur l'une desdites parois latérales 12 du logement, notamment sur la paroi latérale la plus longue.

Comme représenté sur les figures 1 et 2, la poche 1 comporte sur une des parois latérales au moins un orifice d'entrée 13 et/ou de sortie 14,15 du produit biologique. En particulier, tous les orifices d'entrée/sortie 13-15 sont arrangés sur la même paroi latérale 11 afin de réduire au maximum les dimensions de la poche 1.

Notamment, la poche 1 comporte un orifice d'entrée 13 pour l'introduction du produit biologique dans la poche. Cet orifice est relié à l'extrémité d'une tubulure 16. L'autre extrémité de la tubulure est reliée à un ou plusieurs moyens 17,18,19 de connexion à des poches ou des seringues, tels que des connecteurs de type perforateur 17, luer mâle 18, luer femelle ou connecteur auto-obturable sans aiguille 19.

Comme représenté sur la figure 1, la tubulure 16 est également munie d'un site d'injection 20 sous forme d'un luer auto-obturable, pour, par exemple, apporter un additif au produit biologique.

En variante de réalisation, la tubulure 16 comporte un raccord 21 pour connecter des portions de tubulure réalisées dans des matériaux différents. Sur la figure 1, le raccord 21 permet de connecter la portion de tubulure 22 reliée à la poche et réalisée en éthylène vinyle acétate à l'autre portion de tubulure réalisée en polychlorure de vinyle 23.

La tubulure 16 est également pourvue d'un ou plusieurs moyens 24 sélectifs de fermeture et/ou ouverture de la communication fluidique dans la tubulure 16. De tels moyens sont par exemple des clamps ou des ouvre-circuits.

Un orifice de sortie 14,15, distinct de l'orifice d'entrée 13, est avantageusement prévu pour vider le produit biologique après conservation, au moment de son utilisation. L'orifice de sortie 14,15 est formé par une portion de tubulure fermée par une ailette 25,26. Pour accéder à l'orifice 14,15, il suffit d'appliquer un mouvement de rotation à l'ailette 25,26 pour la séparer de la poche.

La poche 1 comporte un ou plusieurs compartiments. Le nombre et la surface des différents compartiments varient selon le volume devant être contenu dans chacun des compartiments définis. Selon une réalisation particulière, la poche 1 comporte au moins deux compartiments 27,28 séparés par une partition 29. La partition est formée par une soudure entre les deux compartiments 27,28. En particulier, la partition 29 est interrompue à un ou plusieurs endroits 30,31 pour permettre une communication fluidique entre les différents compartiments 27,28. Encore plus particulièrement, les interruptions 30,31 se situent aux extrémités de la partition.

En outre, la partition 29 comprend avantageusement une fente 32 pour faciliter la séparation des compartiments 27,28 le cas échéant.

Comme représenté sur la figure 1, chaque compartiment 27,28 comprend un orifice de sortie 14,15, notamment sous la forme d'une portion de tubulure fermée par une ailette 25,26.

Après avoir introduit le produit biologique dans la poche tridimensionnelle 1, la tubulure 16 est fermée et coupée, notamment par soudure, afin de séparer la poche 1 de la portion de tubulure comprenant les connecteurs 17-19 et autres éléments tels que le raccord 21 et le site d'injection 20.

Une portion de tubulure reliée à l'orifice d'entrée de la poche est avantageusement conservée afin de réaliser un échantillonnage du produit biologique contenu dans la poche. Pour ce faire, la portion de tubulure remplie de produit biologique est divisée en deux ou plusieurs segments de tubulure comportant chacun un échantillon de produit biologique. Les segments sont notamment réalisés par soudure.

Le logement 3 de l'enveloppe 2 possède des dimensions suffisantes pour envelopper la poche 1 munie de ses orifices d'entrée 13 et/ou sortie 14,15 et éventuellement de la portion de tubulure servant à l'échantillonnage.

La poche 1 comprend une fente 33 permettant de suspendre la poche 1 lors de la perfusion du produit biologique à un patient. Avantageusement, la fente 33 est disposée en regard du ou des orifices de sortie 14,15 de la poche 1. La fente 33 de suspension est plus particulièrement positionnée sur le joint périphérique de la poche, au niveau d'un élargissement dudit joint.

Sur la figure 1, la poche 1 comprend une étiquette 34 pour assurer l'identification et la traçabilité du produit biologique à conserver.

Le volume interne de la poche est compris entre 1 ml et 1 L, particulièrement entre 5 et 50 ml, et plus particulièrement d'environ 25 ml. Lorsque la poche 1 est destinée à conserver une unité de sang de cordon, son volume interne est compris entre 5 et 50 ml, notamment 25 ml.

Selon une réalisation particulière de l'invention, l'enveloppe 2 est formée d'une première feuille 35 et d'une deuxième feuille 36, au moins l'une des feuilles présentant un relief 37,38 formant au moins une partie du logement 3 pour la poche 1, les deux feuilles 35,36 étant assemblées entre elles le long d'un joint périphérique 39 entourant ledit relief, le joint périphérique 39 présentant une interruption pour former la fente 4 de l'enveloppe 2.

Comme représenté sur la figure 3, le joint périphérique 39 se prolonge sur une partie de la périphérie des bandes 5,6. Avantageusement, le joint périphérique se prolonge sur une partie seulement des bandes 5,6. Ainsi, les bandes 5,6 se séparent facilement pour permettre l'ouverture de la fente 4 et l'insertion de la poche 1. Et la prolongation du joint périphérique 39 sécurise le joint périphérique de l'enveloppe en créant un point de fragilité éloigné du relief 37,38 formant le logement 3.

Le relief 37,38 présente une surface sensiblement plane correspondant à la face antérieure 8 ou postérieure 10 du logement 3 de l'enveloppe 2 et un rebord entourant ladite surface correspondant au moins à une partie des parois latérales 12 du logement 3.

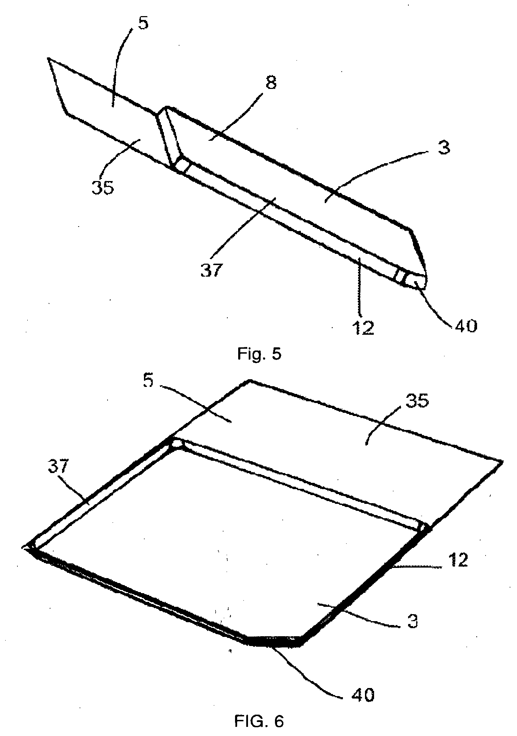

Sur les figures 4 à 6, le rebord est substantiellement perpendiculaire à la surface plane du relief 37. Ainsi, le relief possède une forme de parallélépipède dont chacune des faces est sensiblement rectangulaire. Les.angles entre d'une part les différentes faces latérales formant le rebord et d'autre part les faces formant la surface plane et les faces latérales sont arrondis ou émoussés.

Sur les figures 3 et 5 à 7, l'une des arêtes latérales du relief 37 parallélépipédique présente un chanfrein 40 obtenu en coupant l'arrête de sorte à retirer une partie en forme de prisme triangulaire et de sorte à réduire l'espace entre la poche 1 et le logement 3, lorsque la poche 1 est dans le logement 3 de l'enveloppe 2.

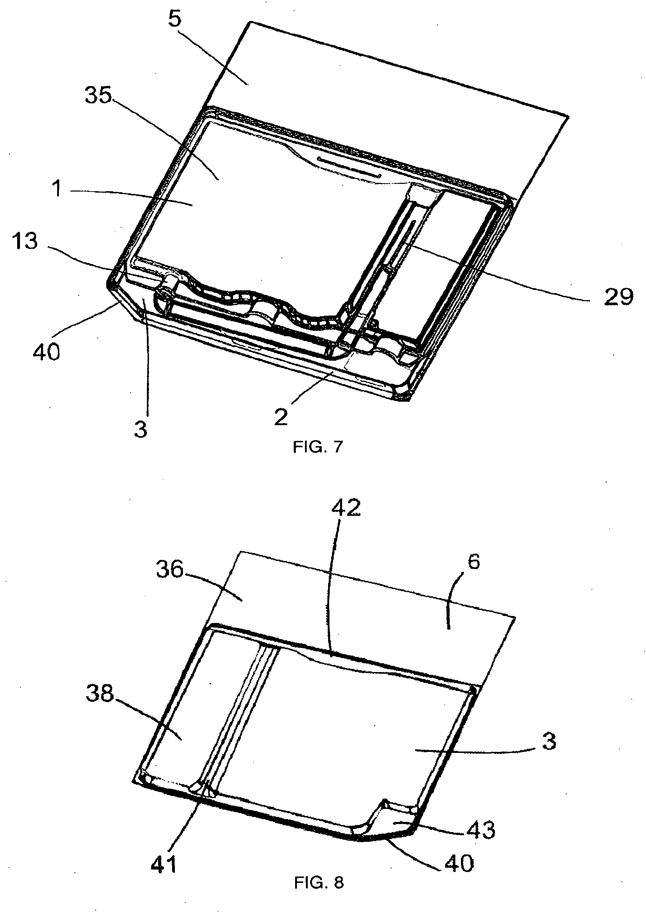

Comme illustré sur la figure 8, le relief 38 de l'une des feuilles 36 au moins comprend une ou plusieurs cavités 41,42,43 correspondant à la géométrie de la poche tridimensionnelle 1.

Par exemple, le relief 38 comprend une cavité 41 correspondant sensiblement à la partition 29 de la poche 1. La cavité 41 se présente sous la forme d'une entaille rectiligne traversant le relief. La cavité est réalisée sur la face correspondant à la face antérieure 8 ou postérieure 10 du logement de l'enveloppe 2 et s'étend entre deux parois latérales opposés du logement 3. La profondeur de l'entaille est égale ou légèrement inférieure à la hauteur du relief 38.

Alternativement ou additionnellement, le relief 38 comprend une cavité 42 semi ovale correspondant à l'élargissement du joint périphérique de la poche, à l'endroit où est disposé la fente 33 de suspension.

Alternativement ou additionnellement et dans l'optique de minimiser encore plus l'espace entre la poche et l'enveloppe, une cavité 43 en forme de triangle est aménagée sur le chanfrein 40 coupée du relief 38.

Ces différentes cavités 41,42,43 rendent les reliefs 37,38 de chacune des feuilles 35,36 dissymétriques, de sorte que la poche 1 ne peut être insérée que dans le sens pouvant faire correspondre les cavités 41,42,43 du relief avec celles de la poche 1. Ainsi, sur les figures, la poche 1 est placée dans le logement 3 de sorte à disposer les orifices 13,14,15 de la poche dans le fond du logement 3 de l'enveloppe 2 , c'est-à-dire à l'opposé de la fente 4 d'insertion.

Selon une première réalisation non représentée sur les figures, seulement une des feuilles comporte un relief. Dans ce cas, l'autre feuille est plane.

Selon une autre réalisation représentée sur les figures, les deux feuilles 35,36 présentent un relief 37,38. Le relief 37,38 de chacune des feuilles est identique ou différent.

Notamment, selon une variante, l'une des feuilles 36 comprend au moins une cavité 41 correspondant à la partition 29 de la poche, et l'autre feuille 35 ne comprend pas de cavité correspondant à la partition de la poche.

L'espace libre entre la feuille 35 et la partition 29 de la poche 1 est alors utilisé avantageusement pour placer, le cas échéant, la portion de tubulure reliée à l'orifice d'entrée 13 de la poche et servant à l'échantillonnage (figure 7).

L'épaisseur de chacune des feuilles 35,36 formant l'enveloppe 2 est comprise entre 0,20 et 0,70 mm, notamment 0,35 mm. Cette épaisseur permet d'obtenir une résistance suffisante, notamment pour éviter une rupture lors de la congélation.

Selon un mode de réalisation, le relief 37,38 de la feuille formant l'enveloppe est réalisé à l'aide d'un moule. Notamment le relief de la feuille formant l'enveloppe est réalisé par thermoformage. D'autres modes de réalisations possibles sont le moulage par extrusion-soufflage ou le moulage sous vide.

En particulier, chacune des deux feuilles 35,36 comprend un relief 37,38 réalisé par thermoformage.

La ou les feuilles thermoformées 35,36 sont réalisées en un matériau thermoplastique souple.

Des matériaux thermoplastiques souples pouvant être thermoformés et soudés sont par exemple l'éthylène acétate de vinyle, le polyéthylène ou fluoropolymère tel qu'un éthylène-propylène fluoré.

Par exemple, l'enveloppe 2 et/ou la poche 1 sont réalisées en éthylène acétate de vinyle, polyéthylène ou fluoropolymère tel qu'éthylène-propylène fluoré.

Notamment, la poche 1 et l'enveloppe 2 sont réalisées dans le même matériau tel que l'éthylène acétate de vinyle.

Les films en éthylène acétate de vinyle possèdent généralement une face lisse et une face légèrement rugueuse. Ce matériau est connu pour adhérer facilement. Afin d'éviter que la poche n'adhère à l'enveloppe, il convient d'éviter de mettre en contact deux faces lisses. Ainsi, il est avantageux de prévoir que la surface extérieure de la poche corresponde à la face lisse du film en éthylène vinyle acétate, et que la surface intérieure de l'enveloppe corresponde à la face légèrement rugueuse du film en éthylène vinyle acétate.

Pour répondre aux exigences sanitaires notamment, la poche et l'enveloppe sont emballées séparément et stérilement. En variante, la poche et l'enveloppe sont emballées ensemble et stérilement.

On décrit ci-après un procédé de conservation d'un produit biologique à l'aide d'un kit selon l'invention. Le procédé comprend les étapes consistant à :
- remplir la poche 1 tridimensionnelle avec le produit biologique à conserver,
- placer la poche 1 ainsi remplie dans le logement 3 de ladite enveloppe 2,
- associer entre elles les bandes 5,6 de l'enveloppe 2 de sorte à fermer hermétiquement le logement 3 de l'enveloppe contenant ladite poche 1,
- stocker la poche 1 placée dans le logement de l'enveloppe dans des conditions adaptées pour conserver ledit produit biologique.

Le remplissage de la poche 1 est réalisé par l'intermédiaire d'un des connecteurs 17,18,19 prévu sur la tubulure reliée à l'orifice d'entrée 13 de la poche 1 ou par connexion stérile de la tubulure 16 avec une autre tubulure reliée à une source de produit biologique.

Comme la poche 1 est tridimensionnelle, elle contient un certain volume d'air qu'il est préférable d'éliminer pour permettre le remplissage de la poche avec le produit biologique à conserver. Pour ce faire, il est possible de connecter une seringue vide à l'un des connecteurs 17,18,19, puis d'aspirer l'air contenu dans la poche à l'aide de la seringue. Une fois cette opération réalisée, le produit biologique peut être transféré dans la poche 1, par gravité notamment.

Le procédé de conservation peut prévoir de mettre un additif tel qu'un agent cryoprotecteur au produit biologique contenu dans la poche. Cet additif est par exemple du diméthyle sulfoxyde. L'ajout de l'additif est réalisé par l'intermédiaire d'un des connecteurs 17,18,19 prévu sur la poche.

Il est avantageux d'éliminer l'air contenu dans la poche avant congélation pour ne pas endommager les cellules lors de la congélation. Cette opération est réalisée à l'aide d'une seringue, en plaçant la poche de telle façon à amener la bulle d'air au niveau de l'orifice d'entrée de la poche. L'air est ensuite aspiré par la seringue.

Pour réaliser l'échantillonnage, le cas échéant, du produit biologique contenu dans la poche, une portion du produit biologique est maintenue dans la tubulure reliée à la poche lors de l'aspiration d'air ou du remplissage de la poche.

Lorsque le produit biologique contenant éventuellement l'additif est dans la poche, la portion de tubulure reliée à l'orifice d'entrée 13 de la poche 1 est soudée et coupée. La portion de tubulure comportant les connecteurs 17,18,19 est jetée. L'autre portion restée liée à la poche 1 est fermée à plusieurs endroits pour former des segments de tubulure, par exemples deux ou trois segments, comportant chacun un échantillon de produit biologique.

La poche remplie est ensuite placée dans le logement 3 de l'enveloppe 2 tridimensionnelle par l'intermédiaire de la fente 4.

Le cas échéant, la tubulure d'échantillonnage est repliée le long de la paroi latérale comprenant les orifices d'entrée/sortie 13,14,15 et dans la partition 29 entre les deux compartiments 27,28.

Pour faciliter l'insertion de la poche 1, il est avantageux de replier une portion de la poche 1 sur elle-même avant l'insertion, puis de faire glisser la poche 1 ainsi pliée dans le logement 3 de l'enveloppe 2 jusqu'au fond dudit logement 3, et enfin de déplier la poche 1 disposée dans le logement 3.

Grâce aux géométries concordantes de la poche 1 et de l'enveloppe 2, et notamment de la ou des cavités 41,42,43 du relief 37,38, la poche 1 vient se placer d'elle-même correctement dans le logement 3 de l'enveloppe 2.

Si l'une des feuilles de l'enveloppe 36 comprend une cavité 41 correspondant à la partition 29 de la poche 1 et pas l'autre feuille 35, on fera attention de disposer la tubulure d'échantillonnage en regard de la feuille de l'enveloppe 35 ne comprenant pas de cavité correspondant à la partition 29 de la poche.

Une fois la poche 1 remplie et en place dans le logement 3, il convient d'associer entre elles les bandes 5,6 de l'enveloppe 2 de sorte à fermer hermétiquement le logement 3 de l'enveloppe 2 contenant ladite poche 1 remplie.

Cette étape de fermeture du logement 3 est réalisée notamment par soudure. Le repérage de l'endroit où la soudure doit être réalisée est aisément identifiable visuellement puisque la fente 4 se situe à l'interface du logement 3 en relief et des bandes 5,6.

En outre, comme l'espace entre la poche 1 et le logement 3 est minime, il n'est pas nécessaire de procéder à une étape de « chasse d'air » avant fermeture de l'enveloppe 2.

L'ensemble poche et suremballage est ensuite stocké dans des conditions adaptées pour conserver ledit produit biologique.

Par exemple, l'ensemble poche et suremballage est disposé dans une cassette métallique avant d'être plongé dans l'azote liquide pour conserver le produit biologique à -196°C.

## Revendications

1. Kit pour la conservation d'un produit biologique comprenant d'une part une poche (1) destinée à contenir ledit produit biologique, ladite poche (1) possédant une géométrie tridimensionnelle sensiblement constante lors de son utilisation, et d'autre part une enveloppe (2) destinée à emballer ladite poche, ladite enveloppe (2) comprenant un logement (3) agencé pour recevoir ladite poche (1), **caractérisé en ce que** ledit logement (3) possède une géométrie tridimensionnelle sensiblement constante lors de son utilisation, et **en ce qu'**une fente (4) est prévue le long d'un bord dudit logement pour permettre l'insertion de la poche (1) dans ledit logement (3), une bande antérieure (5) et une bande postérieure (6) s'étendent extérieurement à partir de ladite fente (4), lesdites bandes étant destinées à être associées entre elles pour fermer hermétiquement ledit logement (3) de l'enveloppe (2).

2. Kit selon la revendication 1, **caractérisé en ce que** les dimensions intérieures de la géométrie dudit logement (3) correspondent sensiblement aux dimensions extérieures de la géométrie de ladite poche (1) à placer dans le logement (3).

3. Kit selon la revendication 1 ou 2, **caractérisé en ce que** ladite poche (1) et ledit logement (3) sont formés respectivement d'une face antérieure (7,8) et d'une face postérieure (9,10) sensiblement planes, reliées entre elles par des parois latérales (11,12), chacune desdites faces et desdites parois de la poche et dudit logement (3) étant destinées à être disposées en regard l'une de l'autre.

4. Kit selon la revendication 3, **caractérisé en ce que** ladite poche (1) comporte sur une des parois latérales (11) au moins un orifice d'entrée (13) et/ou de sortie (14,15) du produit biologique.

5. Kit selon l'une quelconque des revendications 1 à 4, **caractérisé en ce que** la poche (1) comporte au moins deux compartiments (27,28) séparés par une partition (29).

6. Kit selon les revendications 3 à 5, **caractérisé en ce que** ladite fente (4) est prévue sur l'une desdites parois latérales (12) du logement (3).

7. Kit selon l'une quelconque des revendications 1 à 6, **caractérisé en ce que** l'enveloppe (2) est formée d'une première feuille (35) et d'une deuxième feuille (36), au moins l'une des feuilles (35,36) présentant un relief (37,38) formant au moins une partie du logement (3) pour la poche, les deux feuilles (35,36) étant assemblées entre elles le long d'un joint périphérique (39) entourant ledit relief (37,38), le joint périphérique (39) présentant une interruption pour former la fente (4) de l'enveloppe (2).

8. Kit selon la revendication 7, **caractérisé en ce que** le joint périphérique (39) se prolonge sur une partie de la périphérie des bandes (5,6).

9. Kit selon l'une des revendications 6 ou 7, lorsqu'elles dépendent de la revendication 5, **caractérisé en ce que** le relief (37,38) comprend au moins une cavité (41) correspondant sensiblement à la partition (29) de ladite poche.

10. Kit selon l'une quelconque des revendications 7 à 9 **caractérisé en ce que** l'épaisseur de chacune des feuilles (35,36) formant l'enveloppe est comprise entre 0,20 et 0,70 mm.

11. Kit selon l'une quelconque des revendications 7 à 10, **caractérisé en ce qu'**au moins le relief (37,38) d'une des feuilles (35,36) formant l'enveloppe (2) est réalisée par thermoformage.

12. Kit selon la revendication 11, **caractérisé en ce que** chacune des deux feuilles (35,36) comprend un relief (37,38) réalisé par thermoformage.

13. Kit selon la revendication 11 ou 12, **caractérisé en ce que** la ou les feuilles thermoformées (35,36) sont réalisées en un matériau thermoplastique souple.

14. Kit selon l'une quelconque des revendications 1 à 13 **caractérisé en ce que** l'enveloppe (2) et/ou la poche (1) sont réalisées en éthylène vinyl acétate ou polyéthylène ou fluoropolymère.

15. Kit selon l'une quelconque des revendications 1 à 14, **caractérisé en ce que** la poche (1) et l'enveloppe (2) sont réalisées dans le même matériau.

## Patentansprüche

1. Kit zur Konservierung eines biologischen Produkts, einerseits einen Beutel (1) umfassend, der dazu bestimmt ist, das besagte biologische Produkt einzuschließen, wobei der besagte Beutel (1) eine dreidimensionale Geometrie besitzt, die während seiner Verwendung in etwa konstant ist, und andererseits eine Hülle (2), die dazu bestimmt ist, den besagten Beutel zu verpacken, wobei die besagte Hülle (2) ein Gehäuse (3) umfasst, das angeordnet ist, um den besagten Beutel (1) aufzunehmen, **dadurch gekennzeichnet, dass** das besagte Gehäuse (3) eine dreidimensionale Geometrie besitzt, die während ihrer Verwendung in etwa konstant ist, und dadurch, dass entlang eines Randes des besagten Gehäuses ein Spalt (4) vorgesehen ist, um das Einführen des Beutels (1) in das besagte Gehäuse (3) zu ermöglichen, wobei sich jeweils auerhalb vom besagten Spalt (4) ein vorderes Band (5) und ein hinteres Band (6) erstrecken, wobei die besagten Bänder dazu bestimmt sind, miteinander verbunden zu werden, um das besagte Gehäuse (3) der Hülle (2) hermetisch zu verschließen.

2. Kit nach Anspruch 1, **dadurch gekennzeichnet, dass** die Innenabmessungen der Geometrie des besagten Gehäuses (3) in etwa den Außenabmessungen der Geometrie des besagten Beutels (1) entsprechen, der im besagten Gehäuse (3) anzubringen ist.

3. Kit nach Anspruch 1 oder 2, **dadurch gekennzeichnet, dass** der besagte Beutel (1) und das besagte Gehäuse (3) jeweils aus einer Vorderseite (7, 8) und einer Rückseite (9, 10), die in etwa eben sind, gebildet werden, die durch Seitenwände (11, 12) miteinander verbunden werden, wobei jede der besagten Seiten und der besagten Wände des Beutels und des besagten Gehäuses (3) dazu bestimmt sind, einander gegenüber angeordnet zu werden.

4. Kit nach Anspruch 3, **dadurch gekennzeichnet, dass** der besagte Beutel (1) an einer der Seitenwände (11) zumindest eine Eingangsöffnung (13) und/ oder Ausgangsöffnung (14, 15) für das biologische Produkt umfasst.

5. Kit nach irgendeinem der Ansprüche 1 bis 4, **dadurch gekennzeichnet, dass** der Beutel (1) zumindest zwei Abteile (27, 28) umfasst, die durch eine Trennwand (29) voneinander getrennt sind.

6. Kit nach den Ansprüchen 3 bis 5, **dadurch gekennzeichnet, dass** der besagte Spalt (4) an einer der besagten Seitenwände (12) des Gehäuses (3) vorgesehen ist.

7. Kit nach irgendeinem der Ansprüche 1 bis 6, **dadurch gekennzeichnet, dass** die Hülle (2) aus einer ersten Folie (35) und einer zweiten Folie (36) gebildet wird, wobei zumindest eine der Folien (35, 36) ein Relief (37, 38) aufweist, das zumindest einen Teil des Gehäuses (3) für den Beutel bildet, wobei die beiden Folien (35, 36) entlang einer umlaufenden Dichtung (39) miteinander verbunden sind, die das besagte Relief (37, 38) umgibt, wobei die umlaufende Dichtung (39) eine Unterbrechung aufweist, um den Spalt (4) der Hülle (2) zu bilden.

8. Kit nach Anspruch 7, **dadurch gekennzeichnet, dass** sich die umlaufende Dichtung (39) auf einem Abschnitt des Umfangs der Bänder (5, 6) fortsetzt.

9. Kit nach einem der Ansprüche 6 oder 7, wenn sie vom Anspruch 5 abhängig sind, **dadurch gekennzeichnet, dass** das Relief (37, 38) zumindest eine Vertiefung (41) umfasst, die in etwa der Trennwand (29) des besagten Beutels entspricht.

10. Kit nach irgendeinem der Ansprüche 7 bis 9, **dadurch gekennzeichnet, dass** die Stärke einer jeden Folie (35, 36), aus der die Hülle gebildet wird, zwischen 0,20 und 0,70 mm beträgt.

11. Kit nach irgendeinem der Ansprüche 7 bis 10, **dadurch gekennzeichnet, dass** zumindest das Relief (37, 38) einer der Folien (35, 36), aus der die Hülle (2) gebildet wird, durch Warmverformen gebildet wird.

12. Kit nach Anspruch 11, **dadurch gekennzeichnet, dass** jede der beiden Folien (35, 36) ein Relief (37, 38) umfasst, das durch Warmverformen gebildet wird.

13. Kit nach Anspruch 11 oder 12, **dadurch gekennzeichnet, dass** die warmgeformte(n) Folie(n) (35, 36) aus einem flexiblen thermoplastischen Material gefertigt sind.

14. Kit nach irgendeinem der Ansprüche 1 bis 13, **dadurch gekennzeichnet, dass** die Hülle (2) und/ oder der Beutel (1) aus Ethylenvinylacetat oder Polyethylen oder Fluorpolymer gefertigt sind.

15. Kit nach irgendeinem der Ansprüche 1 bis 14, **dadurch gekennzeichnet, dass** der Beutel (1) und die Hülle (2) aus demselben Material gefertigt sind.

## Claims

1. Kit for preserving a biological product comprising on the one hand a bag (1) intended to hold said biological product, said bag (1) having a substantially constant three-dimensional geometry during the use thereof, and on the other hand a casing (2) intended to pack said bag, said casing (2) including a housing (3) arranged such as to receive said bag (1), **characterised in that** said housing (3) has a substantially constant three-dimensional geometry during the use thereof, and **in that** a slot (4) is provided along one edge of said housing in order to enable the bag (1) to be inserted in said housing (3), a front strip (5) and a rear strip (6) extend on the outside away from said slot (4), said strips being intended to be combined with one another in order to hermetically seal said housing (3) of the casing (2).

2. Kit according to claim 1, **characterised in that** the inside dimensions of the geometry of said housing (3) correspond substantially to the outside dimensions of the geometry of said bag (1) to be placed in the housing (3).

3. Kit according to claim 1 or 2, **characterised in that** said bag (1) and said housing (3) are formed respectively of a substantially planar front face (7, 8) and rear face (9, 10), connected together by lateral walls (11, 12), each of said faces and said walls of the bag and of said housing (3) being intended to be arranged facing one another.

4. Kit according to claim 3, **characterised in that** said bag (1) comprises on one of the lateral walls (11) at least one inlet (13) and/or outlet (14, 15) orifice of the biological product.

5. Kit according to any of claims 1 to 4, **characterised in that** the bag (1) comprises at least two compartments (27, 28) separated by a partition (29).

6. Kit according to claims 3 to 5, **characterised in that** said slot (4) is provided on one of said lateral walls (12) of the housing (3).

7. Kit according to any of claims 1 to 6, **characterised in that** the casing (2) is formed from a first sheet (35) and from a second sheet (36), with at least one of the sheets (35, 36) having a relief (37, 38) forming at least one portion of the housing (3) for the bag, with the two sheets (35, 36) being assembled together along a peripheral seal (39) surrounding said relief (37, 38), with the peripheral seal (39) having an interruption in order to form the slot (4) of the casing (2).

8. Kit according to claim 7, **characterised in that** the peripheral seal (39) extends over a portion of the periphery of the strips (5, 6).

9. Kit according to one of claims 6 or 7, when they depend on claim 5, **characterised in that** the relief (37, 38) comprises at least one cavity (41) substantially corresponding to the partition (29) of said bag.

10. Kit according to any of claims 7 to 9 **characterised in that** the thickness of each of the sheets (35, 36) forming the casing is comprised between 0.20 and 0.70 mm.

11. Kit according to any of claims 7 to 10, **characterised in that** at least the relief (37, 38) of one of the sheets (35, 36) forming the casing (2) is carried out by thermoforming.

12. Kit according to claim 11, **characterised in that** each of the two sheets (35, 36) comprises a relief (37, 38) carried out by thermoforming.

13. Kit according to claim 11 or 12, **characterised in that** the thermoformed sheet or sheets (35, 36) are made of a flexible thermoplastic material.

14. Kit according to any of claims 1 to 13 **characterised in that** the casing (2) and/or the bag (1) are made of ethylene vinyl acetate or polyethylene or fluoropolymer.

15. Kit according to any of claims 1 to 14, **characterised in that** the bag (1) and the casing (2) are made of the same material.
